Europäisches Patentamt

European Patent Office

Office européen des brevets

(19)

(11) EP 1 199 669 A2

(12) EUROPEAN PATENT APPLICATION

(43) Date of publication:
24.04.2002 Bulletin 2002/17

(51) Int Cl.⁷: G06F 19/00

(21) Application number: 01118368.8

(22) Date of filing: 27.07.2001

(84) Designated Contracting States:
AT BE CH CY DE DK ES FI FR GB GR IE IT LI LU
MC NL PT SE TR
Designated Extension States:
AL LT LV MK RO SI

(30) Priority: 16.10.2000  US 240747 P
16.10.2000  US 240745 P
05.12.2000  US 730214

(71) Applicant: NEC Research Institute, Inc.
Princeton, New Jersey 08540 (US)

(72) Inventors:
• Miller, Jonathan
Plainsboro, NJ (US)
• Zeng, Chen
Rockville, MD (US)
• Wingreen, Ned S.
Princeton, NJ (US)
• Tang, Chao
West Windsor, NJ (US)

(74) Representative: Betten & Resch
Patentanwälte,
Theatinerstrasse 8
80333 München (DE)

(54) **Method and system for designing proteins**

(57)     The present invention provides a method and system for identifying, designing, and synthesizing proteins and protein backbones. The invention permits the qualitative identification of designable protein configurations and synthesis of protein folds. The method and system involve generating backbone protein configurations using a set of dihedral angle pairs, normalizing the total surface exposure of the configurations; generating a random set of sequences of hydrophobicities with uniform weight on the space of allowed sequences; determining, for each randomly generated sequence, which of the remaining configurations is the ground state; recording a ground-state configuration for each sequence wherein the desirable configurations are those containing the most sequences with that configuration as their ground state and finally,

     synthesizing sequences of amino acids for the desirable configurations.

FIG. 5

**Description**

## CROSS REFERENCES TO RELATED APPLICATIONS

This application claims the benefits of U.S. Provisional Applications, Serial Numbers 60/240,745 and 60/240,747 filed on October 16,2000.

## FIELD OF THE INVENTION

The present invention relates to the field of bioinformatics, and more particularly to a method and system for identifying, designing and synthesizing proteins and protein structures.

## BACKGROUND OF THE INVENTION

Proteins are an essential component of all living organisms, constituting the majority of all enzymes and functional elements of every cell. Each protein is an unbranched polymer of individual building blocks called amino acids. In general, there are 20 different natural amino acids, and each protein is a chain of from 50 to 1,000 amino acids. Hence there is a huge number of possible protein molecules. A simple bacterium will only employ a few hundred distinct proteins, while it is estimated that there are 50,000 distinct human proteins. In each case, the information for all these proteins is encoded in the DNA of every cell of the organism. By convention, the region of DNA coding for a single protein is called a "gene." The machinery of the cell interprets the information in the DNA gene to string together the correct sequence of amino acids to form a particular protein. For natural proteins, the amino-acid sequence can be obtained directly from the sequence of DNA bases (A, C, T, G) in the gene for that protein via a known code.

In order for a protein to perform its function, the chain must fold into a particular structure. Although there is some apparatus in the cell that assists folding, it is generally accepted that the natural folded structure is the minimum free energy state of the protein chain. Hence, the information for both the structure and function of each protein is contained in its sequence of amino acids. However, it has proven difficult to theoretically predict the folded structure from a knowledge of the amino-acid sequence.

Experimentally, the native folded structure of several thousand proteins have been obtained by X-ray crystallographic and/or nuclear magnetic resonance techniques. These methods can often identify the average position in the folded protein of every atom, other than hydrogen, to within 1-2 Angstroms. From this detailed structural information, several general observations about proteins have been made.

First, the overall structure of the folded protein can be well-described in terms of the configuration of the backbone plus the orientations of the various amino-acid side chains. Moreover, the backbone configuration is well characterized by the set of dihedral angles, phi and psi, for each amino acid. The covalent bond lengths and three-atom bond angles are found to vary little among structures. ("Configuration" is defined as the series of phi-psi angles describing the trajectory of a protein backbone through three-dimensional space. The word "structure" is used to describe the full atomic coordinates of a folded sequence including, therefore, the side-chain orientations as well as the backbone configuration.)

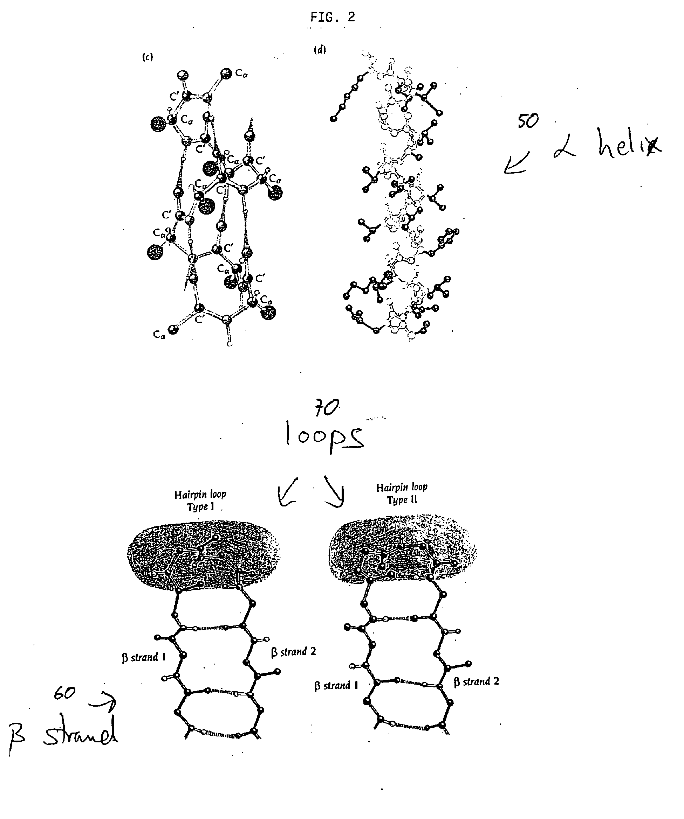

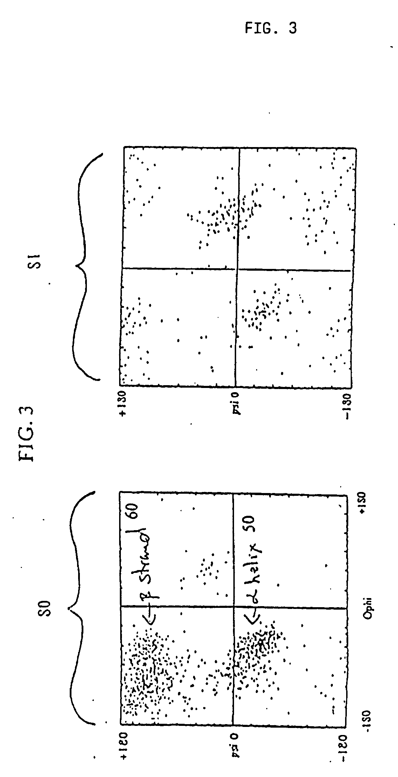

Second, within the natural backbone configurations there is a preponderance of specific motifs or "secondary structures." These are alpha helices (Fig. 2) 50, beta strands (Fig. 2) 60, and loops (Fig. 2) 70. A plot of the frequency of occurrence of particular dihedral angle pairs is called a Ramachandran plot (Fig. 3) 80,81 (from J. Richarson, Adv. Prot. Chem. 34:174-175, 1981). The prevalence of beta strands and alpha helices is clearly indicated by the high frequency of phi-psi pairs in the angular regions associated with these two motifs.

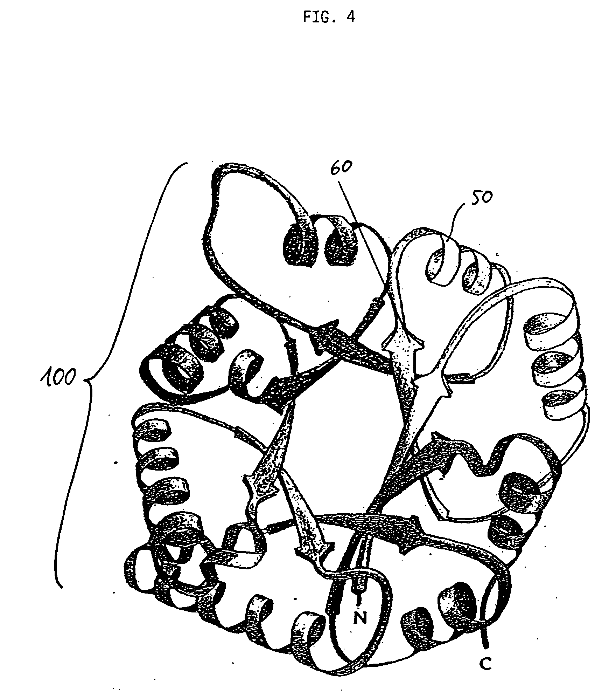

Finally, the secondary structures may be packed together in many different ways. The particular packing of secondary structures is known as the tertiary structure of the protein (Fig. 4) 100. The tertiary structure of two proteins is considered to be the same if both contain the same sequence of secondary structures packed together in the same overall spatial orientation. "Tertiary structure" and "fold" are used interchangeably.

Among the known natural structures, several hundred qualitatively distinct tertiary structures or folds have been identified. Indeed, it has been estimated that there are roughly 2.000 distinct protein folds in nature. Despite the variety of protein sizes, shapes, and backbone configurations represented in the known folding topologies, it remains an open problem to design novel protein folds.

Several protein design techniques have been developed and attempts have been made to develop general design algorithms from these techniques. In designing the modified zinc finger FSD-1, Dahiyat and Mayo used an algorithm for *de novo* protein design (*see B.* Dahiyat et al., "De novo protein design: fully automated sequence selection," *Science,* 1997 Oct 3, 278(5335):82-7). The premise of this algorithm includes the existence and knowledge of a particular backbone configuration with desired characteristics. The backbone configuration chosen was the known backbone of the naturally occurring zinc-finger protein Zif268. The desired characteristics of this backbone were small size and the ability to form an independently folded structure in the absence of disulfide bonds or metal binding.

The algorithm was then applied to the known backbone that tested many possible amino-acid sequences, and many possible side-chain orientations, to find a sequence with particularly low energy when its backbone adopted the exact backbone configuration of Zif268 The low energy sequence would stabilize the backbone chosen.

The structures designed and synthesized by Harbury et al. (P. Harbury et al., "High-resolution protein design with backbone freedom," *Science,* 1998 Nov 20, 282(5393):1462-7) are all coiled coils, i.e. dimers, trimers, or tetramers of alpha helices superhelically twisted about each other. Harbury et al. were able to design sequences of amino acids so that the superhelical twist of these coiled coils was right handed, in contrast to the left handed twist found in nature. The Harbury design algorithm includes determining the hydrophobic-polar residue pattern of a predetermined backbone configuration. Next, is to choose amino acids to pack the core of the chosen pattern. To limit the search for amino acids, only low energy rotamers were considered at each core position. For each core rotamer conformation chosen, mainchain coordinates are determined by exploring a parametric family of backbones. To avoid explicitly modeling unfolded states an energy of permutation is calculated which consists of the difference between two different covalent arrangements of the same amino-acids.

The methods employed by Harbury et al. are very specific to the coiled-coil class of structures. Specifically, only a single family of parametrically related backbone configurations was considered. This backbone configuration was used as a premise to the entire algorithm and the algorithm is inherently dependent on this family of configurations. There is no evident way to generalize this approach to classes of structures other than the coiled coil.

Experimental approaches to designing qualitatively new protein structures have severe limitations. Studies of the folding of random amino acid sequences have identified some sequences which appear to fold. However, the conformations were not sufficiently rigid to allow structural determination by either X-ray crystallography or nuclear magnetic resonance techniques. Without even an approximate knowledge of the folded structure, no systematic progress could be made to increase rigidity.

## SUMMARY OF THE INVENTION

There is no existing method of identifying qualitatively new designable protein configurations. The combination of a method to identify foldable backbone configurations with amino-acid sequence optimization will allow the design and synthesis of qualitatively new protein folds.

A "designable" configuration is one that is the ground state of an unusually large number of amino acid sequences. The sequences associated with designable configurations have protein-like folding properties, i.e. thermodynamic stability, stability under changes of amino acids, and fast folding.

Novel small protein structures offer great promise for the discovery of new drugs. Proteins are generally noncarcinogenic and nonmutagenic, and nontoxic in their breakdown products. Small folded structures may be able to penetrate to drug targets better than larger molecules. New structures imply qualitatively new functions and have the potential for unanticipated medical benefits. Novel small protein structures may also be a source of new antibiotics, pesticides, herbicides, fungicides, etc.

In nature, proteins are also employed in the fabrication of inorganic structures such as bones, teeth, and shells. Proteins have also been employed in nonbiological applications, such as templating of the inorganic synthesis of gold crystallites. Therefore, the new structures enabled by the invention may also allow novel applications of proteins in inorganic synthesis. The ability to design new folds could also prove instrumental in developing methods to predict the folding of natural proteins, the so-called "protein folding problem."

## BRIEF DESCRIPTION OF THE DRAWINGS

Figure 1 depicts the structure of a protein backbone configuration.

Figure 2 depicts common secondary structures occurring within natural backbone configurations.

Figure 3 is a Ramachandran plot.

Figure 4 depicts a tertiary structure or fold.

Figure 5 depicts particular backbone configurations.

Figure 6 is a histogram of designability.

Figure 7 shows graphs depicting particular backbone configurations sensitivity to parameter changes.

Figure 8 is a graph depicting characteristics of a particular backbone configuration.

Figure 9 is a graph depicting inaccessible surface area along the chain in Figure 5 (b) as well as the probability of a hydrophobic amino acid occupying a particular site.

Figure 10 is a graph comparing variance and designability.

## DETAILED DESCRIPTION OF THE PREFERRED EMBODIMENTS

The pattern of surface exposure along the chain is believed to dominate the folding of real proteins. That is, a particular sequence will generally adopt the fold that leaves the hydrophobic ("water fearing") amino acids of the sequence buried in the core of the fold. Therefore, one concentrates on the pattern of surface exposure of each configuration.

The density of protein configurations in a space describing the pattern of surface exposure is slowly varying. This property can be shown to extend to more realistic descriptions of proteins, such as the description based on discrete phi-psi angles described below. Since the overall density of configurations is slowly varying, the distribution of configurations is insensitive to the details of the method by which configurations are generated. Hence, one can identify low density regions of configuration space even from a rough description of possible protein backbone configurations and with a rough approximate energy function.

Configurations in low density regions of configuration space are identified as those configurations which are ground states of a much larger than average number of sequences. "Designability" of a configuration is defined as the number of sequences which have that configuration as their ground state. Those configurations with the highest designability are identified. High designability is a very strong indicator of a low surrounding density of configurations.

The highly designable configurations identified in this way are excellent targets for novel structure design. First, there will be many possible sequences which will fold into these configurations because of the mutational stability of highly designable configurations (this is essentially the definition of high designability). Second, the associated sequences will have few traps, which implies both thermodynamic stability of the ground state and fast folding kinetics. A "trap" is a low energy configuration other than the true ground state. The scarcity of traps follows because it is only configurations with similar patterns of surface exposure that are potential traps for a well-designed sequence. By construction, designable configurations are found in low-density regions of configuration space, which means there are few configurations with similar surface-exposure patterns. Thus all the folding properties normally attributed to real proteins -- mutational stability, thermodynamic stability, and fast folding -- can be associated with those sequences having highly designable ground-state configurations.

One must choose a level of designability below which the configurations are not desirable due to their low possibility of forming stable, compact structures.

The details of the steps of the method of the present invention will now be described.

Step a: Generate large set of compact, self-avoiding backbone configurations:

Step 1a.: Generate backbone configurations using a subset of possible dihedral angles (phi, psi).

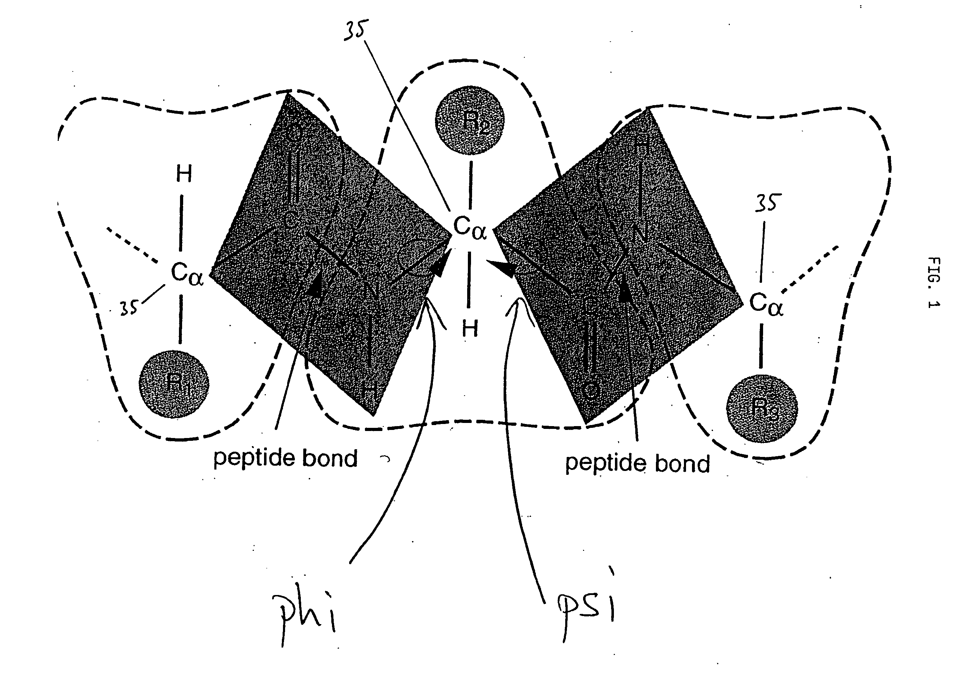

As in figure 1, a small set of specific phi-psi angle pairs, 30, is used to generate a discrete set of backbone configurations. These set the angle and torsion of a peptide bond. If the number of phi-psi pairs is P and the total length of the protein chain is N, then the total number of configurations that can be generated in this way is $P^N$. Typically, at least one set of phi-psi pairs will correspond to an alpha helix, 50, and another to a beta strand, 60, since these two motifs are common in natural protein structures. Or, preferably, two sets of phi-psi pairs will correspond to an alpha helix, 50, and one to a beta strand, 60. Additional pairs should fall within regions of high frequency in the Ramachandran plot, 80, since these represent energetically favorable dihedral angles. The number of angle phi-psi pairs employed will depend on a trade-off between accuracy and computational time: more pairs result in a better sampling of possible configurations, but the larger number of configurations takes more computational time. P = 3 or 4 is preferable.

A method of focusing configurations on designability is by generating structures from a set of dihedral angles, where the probability of choosing a particular pair of dihedral angles depends on the preceding pairs of dihedral angles along the backbone.

Variations of these methods of generating backbone configurations may also be employed. Specifically, one may generate configurations using strings of phi-psi angles. Instead of growing the protein backbone configuration one amino acid at a time, one could add a string of several amino acids at once. This method would differ from the method described above if the various series of phi-psi angles in the strings did not correspond to all possible combinations of a set of individual phi-psi angles. For example, particularly unlikely strings of phi-psi angles could be discarded from the set of strings.

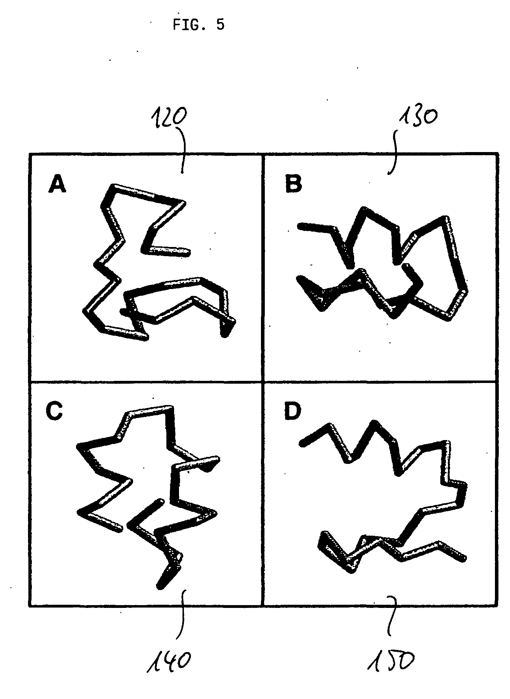

Additionally, instead of generating all configurations allowed by a particular set of phi-psi angles, or strings of phi-psi angles, one can randomly generate a subset of these configurations, and apply the design method described below to this subset. Moreover, by weighting angles or strings according to their frequency of appearance in natural proteins, and possibly allowing strings of different length, one can generate a subset of configurations with statistics of phi-psi angles closely matching the statistics of phi-psi angles in real proteins. Figure 5 shows backbone configurations derived from bond angles favored by natural proteins.

Step 1b.: Eliminate self-intersecting configurations.

If the backbone associated with a particular configuration passes too close to itself in space (self-intersection), then that configuration will be energetically unfavorable for any possible sequence of amino acids. Hence the set of configurations generated in (1a) are eliminated from all those which are self-intersecting. The preferred method to determine if a configuration is self-intersecting is as follows. A sphere of fixed radius to each amino acid in the chain is assigned. The spheres may be centered at the positions of the alpha carbons,35, or, preferably, at a position corresponding to the beta carbon, i.e. the carbon in the side chain which is covalently bonded to the alpha carbon in many amino acids. If two or more spheres overlap, then the configuration is considered to be self-intersecting and is discarded. The radius of the spheres is determined as follows. A representative set of natural backbone configurations is selected. The configurations are decorated with spheres, in the manner described above. The radius employed is the largest radius for which the natural backbone configurations are not self-intersecting.

Step 1c: Eliminate non-compact configurations.

Natural protein configurations are compact. Hence, all those which are not compact are eliminated from the remaining subset of configurations. Compactness is determined by evaluating the total surface exposure of each configuration. A preferred method is as follows. The same assignment of a sphere to each amino acid is made as in (1b). The total surface area is calculated as the surface accessible to a sphere with a radius typical of water molecule. Preferably the method of Flower is employed for this calculation as is known in the art. (See D. Flower, "SERF: a program for accessible surface area calculations," *Journal of Molecular Graphics and Modeling,* 1997 Aug, 15(4): 238-44). If total surface exposure exceeds a particular threshold, the configuration is eliminated. The threshold is set to limit the number of compact structures to a convenient predetermined value.

Step 1d.: Additional criteria.

Additional criteria for configurations can also be applied at this point. For example, only structures with favorable configurations for forming a large number of hydrogen bonds can be retained.

Step 2.: Evaluate designability of remaining backbone configurations.

Step 2a.: Normalize total surface exposure of each configuration.

Different configurations generated above will have different total exposed surface areas, as evaluated in (1c). However, the total surface exposure of each configuration depends on the use of a discrete set of phi-psi angles. Even a slight relaxation of the allowed set of phi-psi angles would allow considerable compactification of the configurations with greatest surface exposure. To remove this artifact of the restricted set of phi-psi angles, a preferred approach is to normalize the total surface exposure of each configuration. In turn, a preferred approach to normalization is to divide the surface exposure of each amino acid in a given configuration by the total surface exposure of that configuration.

Step 2b.: Generate a random set of sequences of hydrophobicities.

For evaluating energies, the method reduces each configuration to its pattern of surface exposure. Similarly, each sequence is reduced to the pattern of hydrophobicities of its individual amino acids. Hydrophobicity is a technical term representing the free energy cost of bringing a particular substance in contact with water. The hydrophobicities of the natural amino acids have been experimentally measured. For purposes of calculating the designability of backbone configurations, the hydrophobicities of the amino acids can be simplified to 0 and 1, or to real numbers between 0 and 1, or one can employ the measured hydrophobicities of natural amino acids. In any case, a set of sequences of hydrophobicities is randomly generated with uniform weight on the space of allowed sequences.

Step 2c. Record the ground-state configuration for the sequence.

Since the designability of a configuration is the number of sequences with that configuration as their ground state, it is necessary to find the ground state of a large number of sequences. A preferred expression for the energy of a sequence folded into a particular configuration is:

$$E = \sum_i h_i \widetilde{a}_i \tag{1}$$

where $h_i$ is the hydrophobicity of the ith element of the sequence and $\tilde{a}_i$ is the normalized surface exposure of the ith amino acid sphere in the particular configuration. For each sequence considered, one must record the configuration with the lowest energy given by the previous equation; that is, one must record the ground-state configuration for that sequence. It is not necessary to find the ground-state configuration for all sequences. By sampling a large number of randomly selected sequences, it is possible to reliably estimate the designabilities of different configurations.

Step 2d.: Sum the designability of all configurations within each cluster.

In the determination of the designability of configurations, those configurations with similar patterns of surface exposure are considered to compete. However, two configurations which are very similar in their total geometry should not be considered as competing folds, but rather as variants of the same fold. Hence, if two configurations are sufficiently similar in the three dimensional trajectory followed by their backbones, then they are considered to be members of a single configuration cluster.
Clustering is preferably carried out as follows. The total root-mean-square distance between every pair of configurations is determined. (The root-mean-square distance between two configurations is the sum of the root-mean-square distances between corresponding alpha carbons along the chains. In performing this calculation, the two configurations are oriented relative to each other in the way which minimizes their root-mean-square distance.) Cluster of configurations are then defined such that a configuration is a member of a cluster if it lies within a root-mean-square distance lambda of any member of the cluster. The distance $\lambda$ is preferably about 0.4 Angstroms per amino acid. Smaller values of $\lambda$ fail to cluster geometrically similar configurations, larger values create very large clusters including dissimilar configurations.
All configurations within a cluster are treated as variants of a single configuration. Therefore, one sums the designabilities of all configurations within each cluster, and consider the total to be the designability of the cluster.
Figure 5 displays the first 120, fourth 130, and 15th 140 most designable structures from a calculation of designability for protein chains of up to $N$=23 amino acids using bond angles favored by natural proteins. One structure 150 from this calculation resembles the natural zinc finger.

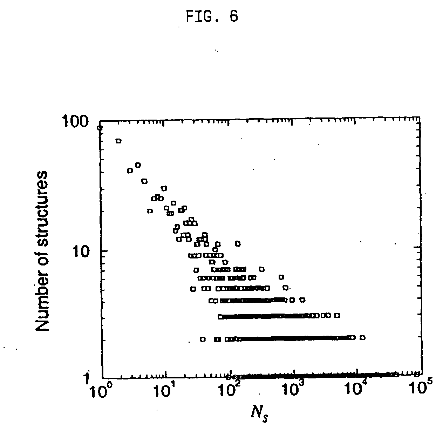

Figure 6 is a histogram of designabilities of 23-mer structures. The surface area cutoff $A_c$ is such that 10,000 configurations are included in the calculation which are grouped into 4688 clusters with the cluster radius $\lambda$ = 0.4Å.

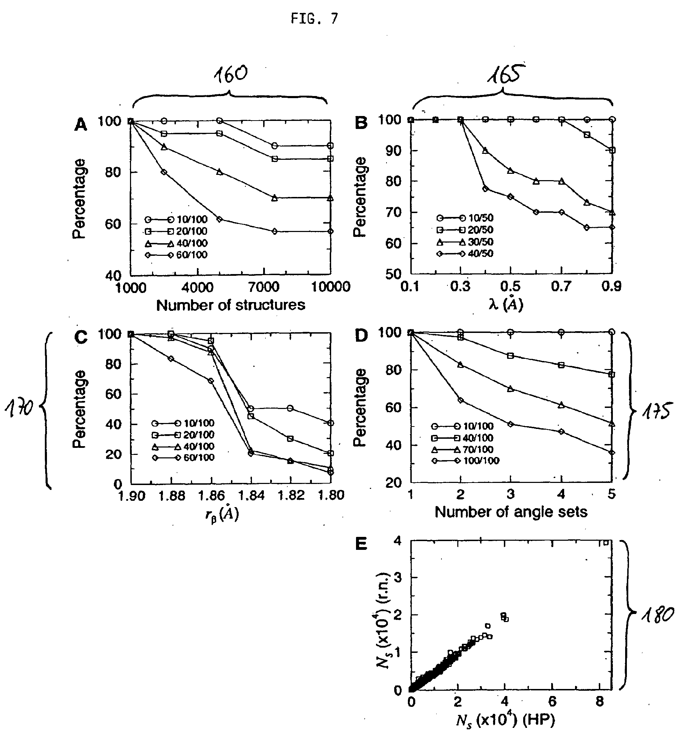

Figure 7 depicts the sensitivity to parameter changes for the top structures of the 23-mer. First 160 is a fraction of the top 10, 20, 40, or 60 most designable structures which remain in the top 100 as the surface-area cutoff is increased. The initial cutoff is chosen so that only the 1,000 most compact configurations are allowed, and is increased until 10,000 configurations are allowed. Second 165 is a fraction of the top 10, 20, 30, or 40 most designable structures which remain in the top 50 as the clustering radius $\lambda$ is increased. The 5,000 most compact configurations are included in the calculation and $r_\beta$=1.9 Å. Third 170 is a fraction of the top 10, 20, 40, or 60 most designable structures which remain in the top 100 as the sidechain radius $r_\beta$ is changed. 5,000 configurations are included in the calculation and $\lambda$ = 0.4Å. Fourth 175 is a fraction of the top 10, 40, 70, or 100 most designable structures which remain in the top 100 as configurations from other angle sets are added. The values of the five angle sets are: Set #1 = (-95°, 135°), (-75°, -25°), (-55°, -55°): Set #2 = (-95°, 135°). (-85°, -55°), (-65°, -25°); Set #3 = (-105°, 145°). (-85°, -15°), (-75°. -35°); Set # 4 = (-105°, 145°), (-85°, -35°), (-85°, -5°): Set #5 = (-105°, 145°), (-85°, -35°). (-85°, -15°). Fifth 180 depicts the designability of structures obtained from 4,000,000 randomly generated sequences of real numbers in [0,1] versus that from the enumeration of HP (binary) sequences. The 10,000 most compact configurations are included in the calculation and $\lambda$ = 0.4 Å.

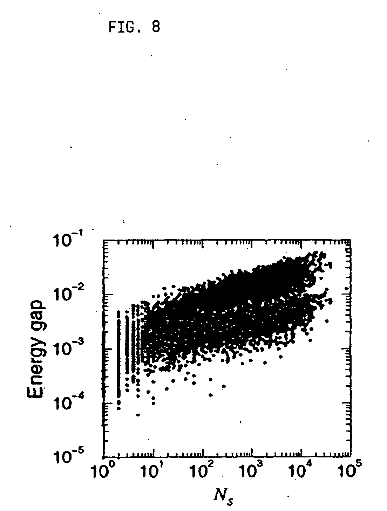

Figure 8 depicts the maximum energy gap (red dots) and average energy gap (black dots) for the sequences which design a given structure, plotted versus structure designability. The 10,000 most compact configurations of the 23-mer are used in the calculation.

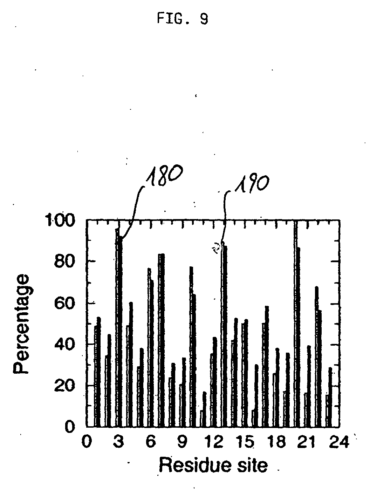

Figure 9 depicts the inaccessible surface for residues ($C_\beta$ spheres) of the highly designable configurations 130 are in the solid bars 180. The probability that each site along the chain is occupied by a hydrophobic amino acid, averaged over all sequences that design the configuration is shown as hollow bars 190.

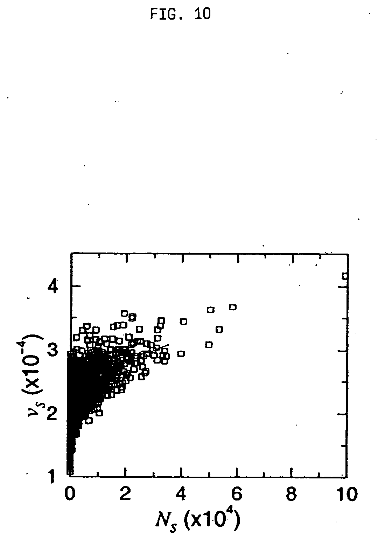

In Figure 10, the average variance vs of a cluster versus the designability $N_S$ of the cluster for the 23-mer. The 5000 most compact configurations are included in the calculation and $\lambda = 0.4$Å along with a running average with bin size 30.

Step 3.: Design sequences of amino acids which will adopt a target backbone configuration.

The target configurations for design of qualitatively new protein structures are those configurations belonging to clusters with the largest cluster designabilities. Sequences designed to fold into these configurations will exhibit excellent, protein-like folding properties.

The relative coordinates of all the constituents of a given amino acid are precomputed. To add a new amino acid to a structure, these relative coordinates are rotated to the reference frame of the current terminal peptide bond. Such a procedure minimized the number of floating point operations required for each addition.

Before a new amino acid is added to the end of a structure, it is checked that no atoms of this new amino acid overlap with atoms of current peptide. If overlap occurs, this branch of the tree is terminated (pruned). Other criteria, such as surface area, number of contacts, radius of gyration, and minimal bounding sphere radius can also employ for pruning when appropriate.

Step 4.: Synthesize sequences of amino acids.

Any method for sequencing amino-acids may be used for this step.

In place of steps "1b" and "1c", or in addition to them, one can identify designability by patterns of surface exposure. One can use a quantity (Variance) as a proxy for designability. Since Variance can be estimated very accurately from a relatively small sample of all structures, it can be used as a predictor of designability.

Each structure "k" has associated with it a normalized string of surface exposure $\tilde{a}_{k,i}$, where $i$ labels the site (or amino acid) along the chain. The Variance of structure $k$ is defined

$$V = \sum_i (\tilde{a}_i - \langle \tilde{a}_i \rangle)^2,$$

where

$$\langle \tilde{a}_i \rangle = 1/\text{Total} \sum_{k'} \tilde{a}_{k'i}.$$

"Total" is the total number of compact, self avoiding structures obtained, so $\langle a_i \rangle$ is the average normalized surface exposure of site i, with the average taken over the structures obtained.

A structure with a high Variance is one in which some amino acids are very well buried in the core and so have very small surface exposure. The remaining amino acids are exposed. A structure with high Variance will typically be highly designable. Any sequences with hydrophobic monomers at sites corresponding to the well buried core of the structure is likely to have that structure as its ground state. This leads to high designability. A structure with a low Variance does not have hydrophobic monomers well buried in the core and has a large surface exposure.

**Claims**

**1.** A method for identifying desirable protein backbone configurations comprising:

generating backbone protein configurations using a set of dihedral angle pairs;
normalizing the total surface exposure of each remaining configuration;
generating a random set of sequences of hydrophobicities with uniform weight on the space of allowed sequences;
determining, for each randomly generated sequence, which of the remaining configurations is the ground state, and;
recording a ground-state configuration for each sequence wherein the desirable configurations are those containing the most sequences with that configuration as their ground state.

2. A method for identifying desirable protein backbone configurations as in claim 1 wherein:

   one pair of dihedral angle pairs corresponds to an alpha helix and one pair of dihedral angle pairs corresponds to a beta strand.

3. A method for identifying desirable protein backbone configurations as in claim 1 wherein:

   two sets of dihedral angles correspond to an alpha helix and one set of dihedral angle pairs corresponds to a beta strand.

4. A method for identifying desirable protein backbone configurations as in claim 3 wherein:

   additional dihedral angles fall within regions of high frequency in a Ramachandran plot.

5. A method for identifying desirable protein backbone configurations as in claim 4 wherein:

   the probability of choosing a particular pair of dihedral angles depends on the preceeding pairs of dihedral angles along the backbone.

6. A method for identifying desirable protein backbone configurations as in claim 5 further comprising:

   eliminating self-intersecting configurations.

7. A method for identifying desirable protein backbone configurations as in claim 6 further comprising:

   eliminating non-compact configurations.

8. A method for identifying desirable protein backbone configurations as in claim 7 further comprising:

   clustering configurations sufficicntly similar in the three dimensional trajectory followed by their backbones and treating all configurations within a cluster as variants of a single configuration, and;
   summing, for all configurations in a cluster, the number of sequences with that configuration as their ground state such that the sum is considered the designability of the cluster.

9. A method for identifying desirable protein backbone configurations as in claim 8 further comprising:

   eliminating configurations with low Variances.

10. A method for identifying desirable protein backbone configurations as in claim 1 wherein:

    the set of dihedral angle pairs is a set of strings of dihedral angle pairs.

11. A method for identifying desirable protein backbone configurations as in claim 10 wherein:

    the strings of angles are weighted according to their frequency of appearance in natural proteins and infrequent strings are eliminated.

12. A method for identifying desirable protein backbone configurations as in claim 1 wherein:

    normalizing is accomplished by dividing the surface exposure of each amino acid in a given configuration by the total surface exposure of that configuration.

13. A method for identifying desirable protein backbone configurations as in claim 1 further comprising:

eliminating configurations with low Variance.

**14.** A method for identifying desirable protein backbone configurations as in claim 1 further comprising:

eliminating self-intersecting configurations.

**15.** A method for identifying desirable protein backbone configurations as in claim 14 further comprising:

eliminating non-compact configurations.

**16.** A method for identifying desirable protein backbone configurations as in claim 1 further comprising:

eliminating non-compact configurations.

**17.** A method for identifying desirable protein backbone configurations as in claim 1 further comprising:

eliminating configurations with low Variance.

**18.** A method for identifying desirable protein backbone configurations as in claim 1 further comprising:

eliminating all configurations that are not favorable for forming a large number of hydrogen bonds after eliminating non-compact configurations.

**19.** A method for identifying desirable protein backbone configurations as in claim 1 further comprising:

clustering configurations sufficiently similar in the three dimensional trajectory followed by their backbones and treating all configurations within a cluster as variants of a single configuration, and;
summing, for all configurations in a cluster, the number of sequences with that configuration as their ground state such that the sum is considered the designability of the cluster.

**20.** A method for identifying desirable protein backbone configurations as in claim 19 wherein:

clustering is accomplished by totaling the root-mean-square distance between every pair of configurations and defining a configpration as a member of a cluster if it lies within a root-mean-square distance $\lambda$ of any member of the cluster.

**21.** A method for identifying desirable protein backbone configurations as in claim 20 wherein:

$\lambda$ is 0.4 Å per amino acid.

**22.** A method for designing proteins comprising:

generating backbone protein configurations using a set of dihedral angle pairs;
eliminating self-intersecting configurations;
normalizing the total surface exposure of each remaining configuration;
generating a random set of sequences of hydrophobicities with uniform weight on the space of allowed sequences for each remaining configuration;
determining, for each randomly generated sequence, which of the remaining configurations is the ground state;
recording the ground-state configuration for each sequence wherein desirable configurations are those containing the most sequences with that configuration as their ground state, and;
synthesizing sequences of amino acids for the desirable configurations.

**23.** A method for designing proteins as in claim 22 wherein:

one set of dihedral angle pairs corresponds to an alpha helix and one set of dihedral angles corresponds to a beta strand.

**24.** A method for designing proteins as in claim 22 wherein:

two sets of dihedral angles correspond to an alpha helix and one set of dihedral angle pairs corresponds to a beta strand.

**25.** A method for designing proteins as in claim 24 wherein:

additional dihedral angle pairs fall within regions of high frequency in a Ramachandran plot.

**26.** A method for designing proteins as in claim 25 wherein:

the probability of choosing a particular pair of dihedral angles depends on the preceeding pairs of dihedral angles along the backbone.

**27.** A method for designing proteins as in claim 26 further comprising:

eliminating self-intersecting configurations.

**28.** A method for designing proteins as in claim 27 further comprising:

eliminating non-compact configurations.

**29.** A method for designing proteins as in claim 28 further comprising:

clustering configurations sufficiently similar in the three dimensional trajectory followed by their backbones and treating all configurations within a cluster as variants of a single configuration, and;
summing, for all configurations in a cluster, the number of sequences with that configuration as their ground state such that the sum is considered the designability of the cluster.

**30.** A method for designing proteins as in claim 29 further comprising:

recording the Variance of each configuration, ranking the configurations from highest Variance to lowest, and designing proteins starting with the configurations having the highest Variance.

**31.** A method for designing proteins as in claim 22 wherein:

the set of dihedral angles is a set of strings of dihedral angles.

**32.** A method for designing proteins as in claim 31 wherein:

the strings of angles are weighted according to their frequency of appearance in natural proteins and infrequent strings are eliminated.

**33.** A method for designing proteins as in claim 22 wherein:

normalizing is accomplished by dividing the surface exposure of each amino acid in a given configuration by the total surface exposure of that configuration.

**34.** A method for designing proteins as in claim 22 further comprising:

recording the Variance of each configuration, ranking the configurations from highest Variance to lowest, and designing proteins starting with the configurations having the highest Variance.

**35.** A method for designing proteins as in claim 22 further comprising:

eliminating non-compact configurations after self-intersecting configurations are eliminated.

**36.** A method for designing proteins as in claim 35 further comprising:

clustering configurations sufficiently similar in the three dimensional trajectory followed by their backbones and treating all configurations within a cluster as variants of a single configuration, and;
summing, for all configurations in a cluster, the number of sequences with that configuration as their ground state such that the sum is considered the designability of the cluster.

**37.** A method for designing proteins as in claim 22 further comprising:

eliminating all configurations that are not favorable for forming hydrogen bonds after eliminating non-compact configurations.

**38.** A method for designing proteins as in claim 22 further comprising:

clustering configurations sufficiently similar in the three dimensional trajectory followed by their backbones and treating all configurations within a cluster as variants of a single configuration, and;
summing, for all configurations in a cluster, the number of sequences with that configuration as their ground state such that the sum is considered the designability of the cluster.

**39.** A method for designing proteins as in claim 38 wherein:

clustering is accomplished by totaling the root-mean-square distance between every pair of configurations and defining a configuration as a member of a cluster if it lies within a root-mean-square distance $\lambda$ of any member of the cluster.

**40.** A method for designing proteins as in claim 39 wherein:

$\lambda$ is 0.4 Angstroms per amino acid.

**41.** A method for analyzing the designability of protein backbone configurations to determine if the number of sequences each configuration has in its ground state is larger than a predetermined number comprising:

generating backbone protein configurations using a set of dihedral angle pairs;
eliminating self-intersecting configurations;
normalizing the total surface exposure of each remaining configuration;
generating a random set of sequences of hydrophobicities with uniform weight on the space of allowed sequences;
determining, for each randomly generated sequence, which of the remaining configurations is the ground state;
recording a ground-state configuration for each sequence wherein the desirable configurations are those containing the most sequences with that configuration as their ground state, and;
comparing how many sequences each configuration has in its ground-state with the predetermined number whereby configurations with larger numbers are highly designable.

**42.** A method for analyzing the designability of protein backbone configurations as in claim 41 wherein:

normalizing is accomplished by dividing the surface exposure of each amino acid in a given configuration by the total surface exposure of that configuration.

**43.** A method for analyzing the designability of protein backbone configurations as in claim 41 wherein:

one set of dihedral angle pairs corresponds to an alpha helix and one set of dihedral angle pairs corresponds to a beta strand.

**44.** A method for analyzing the designability of protein backbone configurations as in claim 41 wherein:

two sets of dihedral angle pairs correspond to an alpha helix and one set of dihedral angle pairs corresponds to a beta strand.

**45.** A method for analyzing the designability of protein backbone configurations as in claim 44 wherein:

additional dihedral angles fall within regions of high frequency in a Ramachandran plot.

**46.** A method for analyzing the designability of protein backbone configurations as in claim 45 wherein:

the probability of choosing a particular pair of dihedral angles depends on the preceeding pairs of dihedral angles along the backbone.

**47.** A method for analyzing the designability of protein backbone configurations as in claim 46 further comprising:

eliminating non-compact configurations after self-intersecting configurations are eliminated.

**48.** A method for analyzing the designability of protein backbone configurations as in claim 47 further comprising:

recording the Variance of each configuration, ranking the configurations from highest Variance to lowest, and designing proteins starting with the configurations having the highest Variance.

**49.** A method for analyzing the designability of protein backbone configurations as in claim 48 further comprising:

clustering configurations sufficiently similar in the three dimensional trajectory followed by their backbones and treating all configurations within a cluster as variants of a single configuration, and;
summing, for all configurations in a cluster, the number of sequences with that configuration as their ground state such that the sum is considered the designability of the cluster.

**50.** A method for analyzing the designability of protein backbone configurations as in claim 41 wherein:

the set of dihedral angle pairs is a set of strings of dihedral angle pairs.

**51.** A method for analyzing the designability of protein backbone configurations as in claim 49 wherein:

the strings of angles are weighted according to their frequency of appearance in natural proteins and infrequent strings are eliminated.

**52.** A method for analyzing the designability of protein backbone configurations as in claim 41 wherein:

the probability of choosing a particular pair of dihedral angles depends on the preceeding pairs of dihedral angles along the backbone.

**53.** A method for analyzing the designability of protein backbone configurations as in claim 41 further comprising:

recording the Variance of each configuration, ranking the configurations from highest Variance to lowest, and designing proteins starting with the configurations having the highest Variance.

**54.** A method for analyzing the designability of protein backbone configurations as in claim 41 further comprising:

eliminating all configurations that are not favorable for forming a large number of hydrogen bonds after eliminating non-compact configurations.

**55.** A method for analyzing the designability of protein backbone configurations as in claim 41 further comprising:

clustering configurations sufficiently similar in the three dimensional trajectory followed by their backbones and treating all configurations within a cluster as variants of a single configuration, and;
summing, for all configurations in a cluster, the number of sequences with that configuration as their ground state such that the sum is considered the designability of the cluster.

**56.** A method for analyzing the designability of protein backbone configurations as in claim 55 further comprising:

clustering is accomplished by totaling the root-mean-square distance between every pair of configurations and defining a configuration as a member of a cluster if it lies within a root-mean-square distance $\lambda$ of any member of the cluster.

**57.** A method for analyzing the designability of protein backbone configurations as in claim 56 further comprising:

$\lambda$ is 0.4 Angstroms per amino acid.

FIG. 1

FIG. 2

(c)

(d)

50

← α helix

70

loops

Hairpin loop
Type I

Hairpin loop
Type II

β strand 1

β strand 2

β strand 1

β strand 2

60 →

β strand

FIG. 3

FIG.3

FIG. 4

FIG. 5

FIG. 6

FIG. 7

FIG. 8

FIG. 9

FIG. 10